# EUROPEAN PATENT APPLICATION

(11) **EP 1 088 889 A1**
(43) Date of publication of application: **04.04.2001**
(21) Application number: 99203209.4
(22) Date of filing: 01.10.1999
(51) Int. Cl.: C12N 15/10, C12N 15/30, C12N 15/49, C12N 15/62, C12N 7/01, C07K 14/16, C07K 14/445, A61K 39/015, A61K 39/21

(54) **Recombinant double hybrid filamentous bacteriophage**

(71) Applicant: UNIVERSITY OF CAMBRIDGE, Cambridge CB2 1QA (GB); Istituto Internazionale di Genetica e Biofisica, 80125 Napoli (IT); Istituto di Biochimica delle Proteine ed Enzimologia, 80125 Napoli (IT); CENTRE NATIONAL DE LA RECHERCHE SCIENTIFIQUE, 75016 Paris Cédex (FR); PASTEUR MERIEUX SERUMS ET VACCINS, 69007 Lyon (FR)
(72) Inventor: Perham, Richard, Cambridge CB3 9NL (GB); Fanutti, Christina, Great Shelford, Cambridge CB2 5LH (GB); Guardiola, John, 80136 Napoli (IT); De Bernardinis, Piergiuseppe, 80125 Napoli (IT); Piatier-Tonneau, Dominique, 75010 Paris (FR); Gaubin, Muriel, 75005 Paris (FR)
(74) Representative: Van Malderen, Joelle

(57) **Abstract**

The present invention is related to a double hybrid filamentous bacteriophage engineering to display at least a CTL epitope and to the pharmaceutical composition comprising said filamentous bacteriophage such as a therapeutical and/or prophylactic vaccine.

### Annex to the application documents - subsequently filed sequences listing

SEQUENCE LISTING

<110> UNIVERSITY OF CAMBRIDGE
      ISTITUTO INTERNAZIONALE DI GENETICA E BIOFISICA
      ISTITUTO DI BIOCHIMICA DELLE PROTEINE ED ENZIMOLOG
      CENTRE NATIONAL DE LA RECHERCHE SCIENTIFIQUE
      PASTEUR MERIEUX SERUMS ET VACCINS

<120> RECOMBINANT DOUBLE HYBRID FILAMENTOUS BACTERIOPHAGE

<130> P.MERI.01/EP

<140>
<141>

<160> 2

<170> PatentIn Ver. 2.1

<210> 1
<211> 8
<212> PRT
<213> Human immunodeficiency virus type 1

<400> 1
Ile Leu Lys Glu Pro Val His Gly Val
  1               5

<210> 2
<211> 15
<212> PRT
<213> Human immunodeficiency virus type 1

<400> 2
Lys Asp Ser Trp Thr Val Asn Asp Ile Gln Lys Leu Val Gly Lys
  1               5                  10                  15

SEQUENCE LISTING

<110> UNIVERSITY OF CAMBRIDGE
ISTITUTO INTERNAZIONALE DI GENETICA E BIOFISICA
ISTITUTO DI BIOCHIMICA DELLE PROTEINE ED ENZIMOLOG
CENTRE NATIONAL DE LA RECHERCHE SCIENTIFIQUE
PASTEUR MERIEUX SERUMS ET VACCINS

<120> RECOMBINANT DOUBLE HYBRID FILAMENTOUS BACTERIOPHAGE

<130> P.MERI.01/EP

<140>
<141>

<160> 2

<170> PatentIn Ver. 2.1

<210> 1
<211> 8
<212> PRT
<213> Human immunodeficiency virus type 1

<400> 1
Ile Leu Lys Glu Pro Val His Gly Val
1               5

<210> 2
<211> 15
<212> PRT
<213> Human immunodeficiency virus type 1

<400> 2
Lys Asp Ser Trp Thr Val Asn Asp Ile Gln Lys Leu Val Gly Lys
1               5                   10                  15

SEQUENCE LISTING

<110> UNIVERSITY OF CAMBRIDGE
      ISTITUTO INTERNAZIONALE DI GENETICA E BIOFISICA
      ISTITUTO DI BIOCHIMICA DELLE PROTEINE ED ENZIMOLOG
      CENTRE NATIONAL DE LA RECHERCHE SCIENTIFIQUE
      PASTEUR MERIEUX SERUMS ET VACCINS

<120> RECOMBINANT DOUBLE HYBRID FILAMENTOUS BACTERIOPHAGE

<130> P.MERI.01/EP

<140>
<141>

<160> 3

<170> PatentIn Ver. 2.1

<210> 1
<211> 8
<212> PRT
<213> Artificial Sequence

<220>
<223> Description of Artificial Sequence: pep789

<400> 1
Ile Leu Lys Glu Pro Val His Gly Val
  1               5

<210> 2
<211> 15
<212> PRT
<213> Human immunodeficiency virus type 1

<400> 2
Lys Asp Ser Trp Thr Val Asn Asp Ile Gln Lys Leu Val Gly Lys
  1               5                  10                  15

<210> 3
<211> 9
<212> PRT
<213> Artificial Sequence

<220>

<223> Description of Artificial Sequence: pep789

<400> 3
Cys Thr Leu Ser Thr Val Gln Leu Val
 1               5

## Description

### Field of the invention

The present invention is related to a recombinant double hybrid filamentous bacteriophage displaying epitopes capable of eliciting an immune response against infectious agents or hyper-proliferative disorders.

The present invention is also related to a pharmaceutical composition comprising an adequate pharmaceutical carrier or diluent and said filamentous bacteriophage, preferably said pharmaceutical composition, is a therapeutical and/or prophylactic vaccine.

### Background of the invention

Protective immunity to viral infections is mediated by neutralising antibodies and by CD8⁺ cytotoxic T lymphocytes (CTLs), both of which depend on antigen specific CD4⁺ T helper (Th) lymphocytes. Several strategies are currently underway to find out preparations which can overcome the risks of recombinant live vectors, such as attenuated virus, vaccinia virus or bacteria and which can bypass the requirement of adjuvants to elicit an efficient response against protein and peptide antigens.

Synthetic peptides carrying epitopes recognised by helper or cytolytic T cells may represent the basis of safe vaccines (1). The development of such subunit vaccines, however, suffers from a number of important limitations. Synthetic peptides are poor immunogens and exhibit short life spans both in serum and intracellularly (2). The amount of peptide required to bind a threshold number of MHC class II molecules on the surface of a presenting cell is reportedly higher than the minimal amount needed to bind to MHC class II in the acidic compartment during processing of the corresponding internalised native antigen (3). Furthermore, peptides carrying CTL epitopes not only present the same drawbacks, but are also inefficiently generated by intracellular processing, because soluble antigens generally do not intersect the appropriate MHC class I compartment. In addition, co-expression of linked helper and CTL epitopes on the surface of the same presenting cell is a strict requirement for priming of a CTL response (4). Delivery systems based on immune-stimulating complexes (ISCOMs), liposomes and synthetic lipopeptides have been designed in an attempt to circumvent these limitations (1).

New technologies based on displaying peptide on the surface of filamentous bacteriophage virions, initially developed to generate peptide libraries (Parmley and Smith, 1988; Smith, 1985), have become important means of raising anti-peptide antibodies (40). The display of specific peptides on the surface of the filamentous recombinant bacteriophage thus prompted the idea of using immunogenic peptides to explore the immune response and to design new types of vaccine. It has been demonstrated that antigenic peptides of 6-8 amino acids can be inserted in the N-terminal region of the major coat protein, gVIIIp, of the filamentous bacteriophage fd, generating a recombinant virion in which all copies of gVIIIp (2700 per virion) display the peptide (5, 8, 40). In order to accommodate larger peptides, it has proved necessary to construct an hybrid phage in which the modified gVIIIp proteins are interspersed with wild-type gVIIIp molecules (5, 34, 25). The inserted peptides, although expressed in a lower copy number on the phage particles, were able to elicit a strong antibody response in various animals (5, 8, 40). Evenmore, it has been shown that immunisation of mice with hybrid phage fd displaying a peptide representing the immunodominant epitope of gpl20 from HIV-1 in the absence of adjuvant can evoke high titres of neutralising antibodies against HIV-1 (7). Studies by NMR spectroscopy have confirmed that the epitope structure, when expressed on filamentous phage virions, can adopt a persistent three-dimensional structure closely resembling that which it exhibits in the wild-type parent protein (35). More recently, the construction of double hybrid bacteriophages has allowed simultaneous display of different peptides on the surface of phage *fd* (26), raising the possibility to display selected Th, CTL and B epitopes in different combinations and to be used as a powerful vaccine vector.

The T-lymphocyte response to an antigen is governed by the source of that antigen and the way in which it is processed. Before recognition by T lymphocytes, proteins must be degraded into peptides which are loaded on major histocompatibility complex (MHC) molecules in antigen-presenting cells (APCs). Two pathways exist within vertebrate cells to generate peptides. The "endogenous" pathway provides peptides to MHC class I molecules for presentation to CD8⁺ CTLs. These peptides usually result from the processing of endogenous intracellular proteins (32). They are loaded in the endoplasmic reticulum (ER) onto newly synthesised MHC class I molecules and transported by the default exocytic pathway to the plasma membrane (38, 42, 43) . The "exogenous" pathway provides peptides to MHC class II molecules for presentation to CD4⁺ Th lymphocytes (3). Such peptides are usually derived from antigens internalised into the endocytic/lysosomal pathway while newly synthesised MHC class II molecules are targeted to endocytic compartments via their association with the invariant chain (Ii). Loading of peptides occurs at an endosomal/lysosomal site and is catalysed by HLA-DM molecules (27, 33, 41).

Hybrid phage *fd*23 displaying a Th epitope, p23 (residues 249-263) from HIV-1 reverse transcriptase (RT) are recognised by human CD4⁺ T cell lines and T cell clones specific for this epitope (28, 29).

### State of the art

The document WO92/07077 describes filamentous bacteriophages including, in at least a proportion of its major coat protein, a foreign peptide of at least six amino acids which is antigenic or immunogenic and is capable of eliciting a biological response, preferably against malaria.

The document WO95/05454 describes the same filamentous bacteriophage engineered to display T cell epitopes and B cell epitopes and/or peptides capable of eliciting HIV neutralising antibodies.

The publication of Malik P. and Perham R. *(Nucleic Acid Research,* Vol. 25, n° 4 (1997), p. 915-916) describes the preparation of double hybrid bacteriophages showing simultaneous display of different peptides on their surface.

This document describes a system for creating hybrid virions, in which the capsid consists of a mixture of the wild type virus sequence and two modified coat proteins carrying different peptide inserts.

Said hybrid virions are obtained from vectors with DNA fragments containing a modified gene VIII encoding foreign peptides T1 and p-66.

Said plasmid vectors were used to transform cells infected with bacteriophages which allow production of virions displaying simultaneously the two different peptides.

This document states also that the displaying of two different peptides simultaneously offers new possibilities of assembling interacting peptides on the surface of the virion and has potential advantages in protein engineering in the study of protein-protein interaction and exploring vaccine design and the immune response. For instance, it is proposed that two different epitopes from a pathogen or an effector sequence and a targeting sequence can be displayed on the same capside.

However, none of these documents have described that it is possible to incorporate into said filamentous bacteriophage a CTL epitope which may induce an (CD8+ cytotoxic T-lymphocytes) immune response.

### Aims of the present invention

The present invention aims to provide a new vector system incorporating foreign epitopes capable of improving an immune response in a patient, against infectious agents or an hyperproliferative disorder, especially mediated by CD8⁺ cytotoxic T lymphocytes (CTLs) and by neutralising antibodies, both of which depend on antigen specific CD4⁺ T helper (Th) lymphocytes.

Another aim of the present invention is to provide such a vector which do not require or reduce the content of adjuvants for a vaccination.

### Summary of the invention

The inventors have discovered that it is possible to incorporate in a double hybrid bacteriophage a CTL epitope capable of eliciting an *in vitro* immune response in a patient and which results advantageously in a protective immunity against infectious agents or against an hyperproliferative disorder.

Furthermore, by the incorporation of a second epitope in said double hybrid bacteriophage, preferably a Th/B epitope, it is also possible to induce by the same engineered double hybrid bacteriophage the activation of CD4⁺ T cell line and Th cell clone specific for this epitope in the patient.

The inventors have discovered that a vaccine comprising said double hybrid bacteriophage before recognition by T lymphocytes is degraded into peptides which are loaded on the major histocompatibility complex (MHC molecules) in an antigen presenting cell (APCs) through the endogenous pathway which provides said exogenous peptides to MHC class I molecules for presentation to CD8⁺ CTLs.

The first aspect of the present invention is related to a double hybrid filamentous bacteriophage engineered to display at least a CTL epitope.

Preferably, the filamentous bacteriophage according to the invention is engineered to further display either a Th cell epitope or a Th cell and B cell epitope.

The incorporation of a Th and B epitope in the double hybrid bacteriophage according to the invention will induce a protective immunity mediated by neutralising antibodies (induced by the B cell epitope) and by CD8⁺ cytotoxic lymphocytes (CTLs) (induced by the CTL epitopes), both of which depend on the antigen specific CD4⁺ Th helper lymphocytes (induced by the Th epitope).

Therefore, the protective immunity against said infectious agents or hyperproliferative disorder is a complete humoral and cellular immune response.

The filamentous bacteriophage according to the invention is engineered in the major coat (pVIII) protein. As the foreign peptides are highly expressed in the pVIII major coat protein (approximately 2700 copies per virion), it is possible to obtain rapidly an effective immune response and a protective immunity against infectious agents and hyperproliferative disorders, without requiring the usual adjuvants used for a therapeutical and/or prophylactic vaccination.

The term "infectious agents" include viral or bacterial agents (i.e. chlamydia, mycobacteria, helicobacter, pylori, etc.) and infectious parasites like plasmodium and tripanosoma.

Preferably, the viral agents are selected from the group consisting of the following infectious viral agents HIV, HTLV, HSV, CMV or HPV and other similar viruses.

An hyperproliferative disorder means the different tissue carcinogenesis and various developments of tumoral cells in a patient.

Preferably, the first epitope incorporated in the engineered filamentous bacteriophage is the CTL epitope RT2 (residues 309-317) having the following amino acid sequence SEQ ID 1:ILKEPVHGV, which is a cytolytic HLA-A2 restricted peptide from HIV 1 reverse transcriptase (RT) (9) .

Preferably, the second epitope is a Th epitope P23 from HIV-1 RT which is able to elicit an anti-HIV-1-CTL response specific for the RT2 CTL epitope (10, 11) .

The PEP 23 corresponds to the immuno dominant 249-263 sequence of RT, having the following amino acid sequence SEQ ID 2 KDSWTVNDIQKLVGK. Other CTL epitopes can be introduced in the bacteriophage according to the invention (i.e. a wild type or modified epitope of the sequence GAG or NEF from HIV viruses).

Other specific (CTL, Th and B) epitopes capable of eliciting an immune response against infectious agents and an hyperproliferative disorder can be selected by the person skilled in the art in order to obtain the required immune response in a patient.

Another aspect of the present invention is related to the pharmaceutical composition comprising an adequate pharmaceutical carrier or diluent (i.e. saline) (and possibly a T cell adjuvant such as QS21 or demethylated CpG oligonucleotides) and said engineered double hybrid filamentous bacteriophage.

Preferably, said pharmaceutical composition is a therapeutical and/or prophylactic vaccine which may possibly require the addition of various adjuvants (including a simple hybrid or a wild type filamentous bacteriophage) .

The pharmaceutical acceptable carrier (vehicle or excipient or diluent) may vary according to the mode of administration and is possibly combined with an adjuvant in order to improve the therapeutical properties of the pharmaceutical composition according to the invention or reduce its possible side effects.

Suitable pharmaceutical acceptable carriers used in the composition according to the invention are well known by the persons skilled in the art and are selected according to the method generally applied by pharmacists and may include solid, liquid or gaseous non-toxic pharmaceutically acceptable carriers.

The percentage of active product/pharmaceutically acceptable carrier may vary within a very large range limited by the tolerance and the possible side effects upon the patient and by the frequency and the mode of administration.

For HLA-A2 transgenic mice, the pharmaceutical composition is administrated subcutaneously with about 150 *µ*g of bacteriophage preparation as described in the following examples.

A corresponding administration to a human will be about 0.2 to 2 mg per injection with 3 injections per individual.

The last aspect of the present invention is related to the use of the recombinant double hybrid filamentous bacteriophage according to the invention for the manufacture of a medicament in the treatment and/or the prevention of infections and a hyperproliferative disorder, preferably infections induced by viral, bacterial and infectious parasites like the plasmodium and tripanosoma.

The present invention is also related to a method of treatment and/or prevention of infections or a hyperproliferative disorder comprising the step of administration a sufficient amount of the pharmaceutical composition to a patient, including the human, in order to induce an immune response, preferably a protective immunity against said infectious agents or said hyperproliferative disorder by viral, bacterial and infectious parasites like plasmodium or tripanosoma.

The present invention will be described in details in the following examples, in reference to the enclosed figures.

### Brief description of the figures

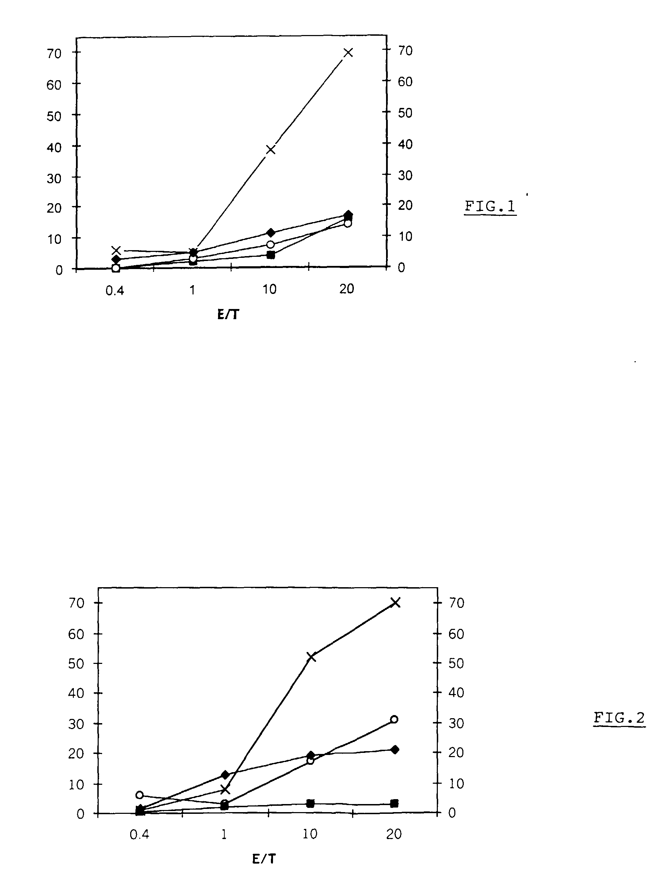

Fig. 1 represents the cytotoxic activity of T cell lines generated from a healthy donor (LDA) HLA A2⁺. 3x10⁵ adherent cells were pulsed for 3 hours with the following antigens: no antigen (■), peptide pRT2 (○), phage single display fdRT2 (◆) , phage double display fd23/RT2 (x). To each of these cultures 1x10⁶ non-adherent cells were added. Cytotoxicity was analysed after 9 days, using as target cells ⁵¹Cr labelled T2 cells pulsed with peptide pRT2. Non specific RT2 lysis was 13% at E/T 20:1. Each experimental point represents the average of three determinations. The set of experiments shown in the Figure is typical of multiple independent CTL priming assays (at least three).

Fig. 2 represents the priming of specific CTL response which requires coexpression of RT2 and pep23 determinants onto the surface of the same presenting cell. Specific cytotoxic activity was induced when adherent cells were simultaneously pulsed with a mix of phages single display fd23 and fdRT2 (x). In contrast levels of cytotoxicity were lower when APC were separately pulsed with phages single display fd23 and fdRT2 and then combined together (○). Moreover, pulsing with synthetic peptides p23 and RT2 (◆) was less efficacious in generating cytotoxic T cells. Control (■) represents culture in the absence of antigen. Non specific T2+ pep-RT2 lysis was <20%. Each experimental point represents the average of three determinations. The set of experiments shown in the Figure is typical of multiple independent CTL priming assays (at least three).

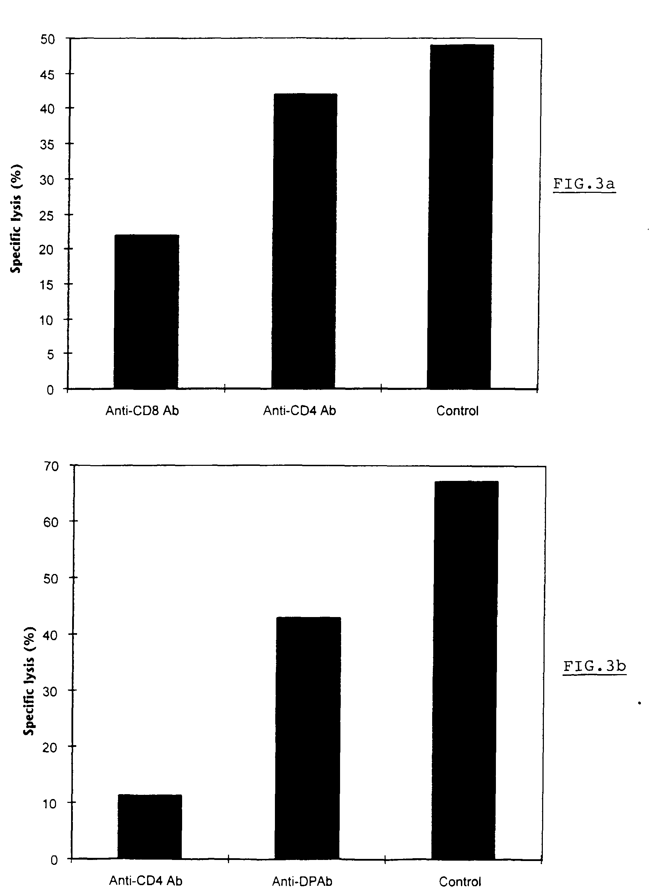

Fig. 3 shows the effect of anti-CD4 and anti-CD8 mAbs on the induction of cytotoxic activity.
**(a)** Effect of anti-CD8 or anti-CD4 mAb on cytotoxic activity of a cell line raised with double display fd23/RT2 phages. Anti-CD8 or anti-CD4 antibody were added at the concentration of 10 µg/ml to mixture of T2 prepulsed target cells and effector cells at the beginning of cytotoxic assay. ⁵¹Cr release at Effector/Target ratio of 20:1 is reported.
**(b)** Effect of anti-CD4 mAb on generation of cytotoxic T cells. Primary cultures pulsed with double display fd23/RT2 phage particles were established in the presence of anti-CD4 mAb or in the presence of an irrelevant control mAb of the same isotype (anti human DQ, Becton Dickinson). The anti-CD4 mAb impaired the induction of cytotoxic T cells as compared with a T cell line raised in the absence of antibody or in the presence of an irrelevant mAb. ⁵¹Cr release at Effector/Target ratio of 30:1 is reported. Non specific target lysis was <20%.

Each experimental point represents the average of three determinations and each experiment was reproduced at least twice.

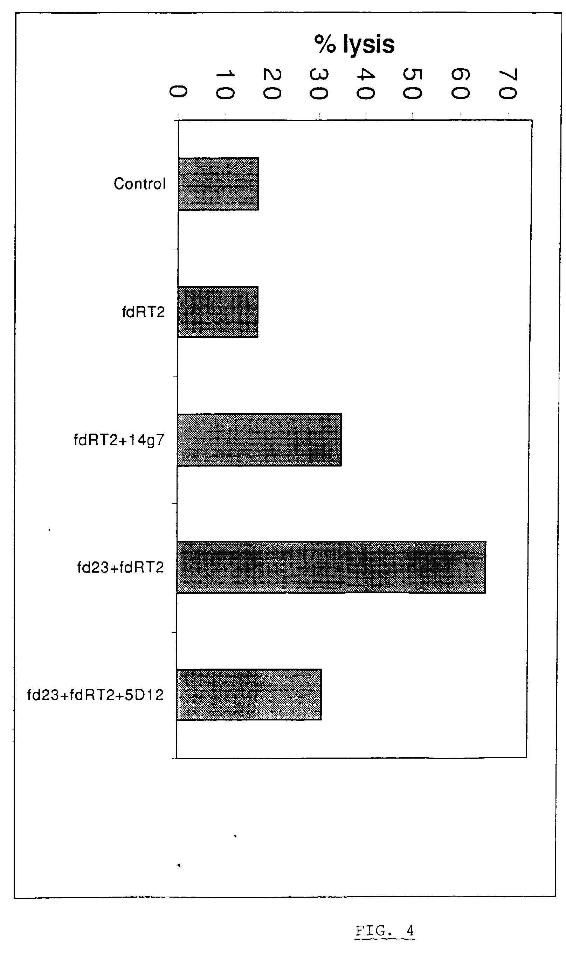

Fig. 4 shows the effect of anti-CD40 mAbs on induction of CTL.

Inhibition of lytic activity of T cells primed with the anti-CD40 blocking monoclonal antibody 5D12. The mAb (19) was added at 10 µg/ml to APC simultaneously pulsed with phage preparations carrying helper and cytolytic epitopes (fd23+fdRT2). Activation of lytic activity of T cells primed with an anti-CD40 activating mAb 14g7. The mAb was added at 10 µg/ml to APC primed with fdRT2. ⁵¹Cr release at Effector/Target ratio of 30:1 is reported.

Each experimental point represents the average of three determinations and each experiment was reproduced at least twice.

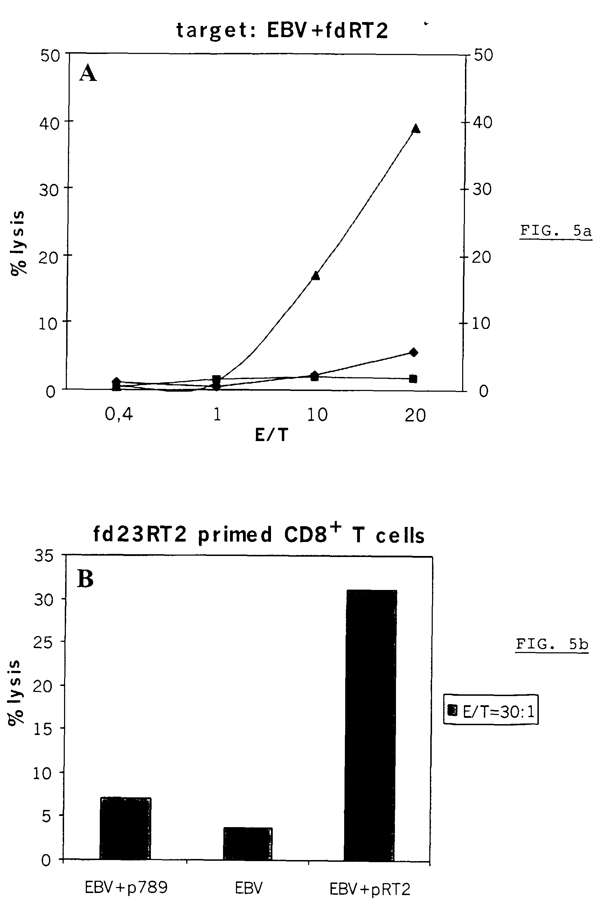

Fig. 5 shows that target EBV-B cell lines pulsed with hybrid phage fdRT2 expressing the CTL epitope RT2 correctly process RT2 and are lysed by specific CTL.
**(a)** cytolytic activity of T cells primed with double hybrid phage fd23/RT2 (▲) , hybrid phage fd23 (◆) and wild-type phage (■) against ⁵¹Cr labelled B-EBV cells pulsed with hybrid phage fdRT2. Results are expressed as the percentage of lysis.
**(b)** cytotoxicity assay using as target B-EBV cells pulsed with the RT2 synthetic peptide (pRT2) and an irrelevant peptide known to bind HLA-A2 (p789). Unpulsed B-EBV cells are used as negative control (EBV).

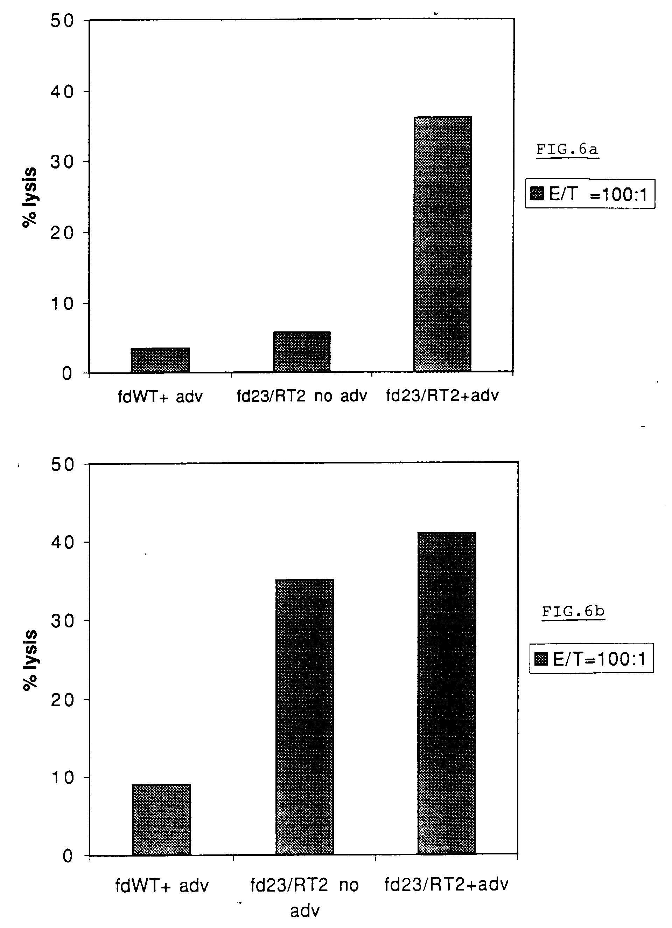

Fig. 6 shows the cytotoxic activity of splenocytes isolated from HLA-A2 transgenic mice which have been immunised with hybrid phage molecules.
**(a)** specific cytotoxic activity of splenocytes towards RMA^{-S} target cells transfected with HLA-A2 molecules. Splenocytes isolated from animals subjected to 1 immunisation with phage particles in the presence or absence of Freund's adjuvant were used as effector cells. E/T ratio was 100:1. x axis: percent lysis. y axis: hybrid molecules used for immunisation.
**(b)** cytotoxic activity of splenocytes isolated from mice after 3 immunisations. E/T: 100:1. x and y axis: as in panel A.

Each experimental point represents the average of three determinations and each experiment was reproduced at least twice.

### Detailed description of the invention

The inventors have developed a delivery system based on the use of a multiple display filamentous *fd* phage. In this frame, a carrier introducing HIV-1 reverse transcriptase-derived B, T-helper and T-cytotoxic epitopes into the highly expressed (approximately 2700 copies per virion) gVIIIp major coat protein has been made. The ability of phages to elicit the production of specific antibodies against a displayed epitope or to present a T-helper epitope has already been documented (5-7). Phage particles can be also addressed to the MHC class I compartment, thus leading to efficient co-presentation of phage-borne helper and CTL epitopes and to helper-dependent priming of a cytolytic T-cell response.

*In vitro* immunisation of human PBL with double hybrid phage fd23RT2 displaying a CTL epitope, RT2 (residues 309-317), and the Th epitope p23 from HIV-1 RT elicited an anti-HIV-1 CTL response specific for the RT2 CTL epitope and that CTL response required RT2 to be displayed by *fd* together with a Th epitope.

A study by means of confocal laser microscopy of the uptake and processing of wild-type filamentous bacteriophage fd particles using as APC an EBV-transformed B cell line (EBV-B) derived from the same donor as the one used for functional studies has been made. Internalised fd phage can be directed to both MHC class I and class II peptide loading compartments in EBV-B cells. In a previous study, it has been demonstrated that B cell lines fed with the hybrid phage fd23 were able to process and present the p23 antigen to CD4⁺ antigen specific T cell lines (De Bernardinis et al 1999). EBV-B cells incubated with *fd* phages expressing the cytolytic epitope RT2 are lysed by specific CTL, which confirms that filamentous phage particles are correctly processed and offer powerful means of recruiting Th and CTL cells which are the basis of an effective vaccine.

### Example 1

### Construction and purification of hybrid bacteriophage

Single display fdp23 and fdRT2 bacteriophages were constructed by ligating a SacII-StuI oligonucleotide insert encoding peptide p23 or pRT2 into *Sac*II-*Stu*I digested plasmid pfd8SHU ⁽²⁴⁾ to generate plasmids pfd8p23 and pfd8pRT2.

*E. coli* TG1 *recO* cells transformed with pfd8p23 or fd8pRT2, were infected with the bacteriophage fdISPLAY8 (24, 25). Double display fdp23/RT2 bacteriophage was constructed by ligating a SacII-StyI oligonucleotide insert encoding peptide pRT2 into SacII-StyI digested bacteriophage fdAMPLAY88 (26) to generate fdAMPLAY88-RT2. The simultaneous display of p23 and RT2 was achieved by infecting *E. coli* TG1 / *recO* cells transformed with pTfd8p-66 (26) plasmid with fdAMPLAY88-RT2. The hybrid bacteriophages were harvested and analysed by SDS-PAGE (25). The number of copies of the protein carrying the inserts relative to the wild-type coat protein was estimated from the relative yields of the N-terminal sequences (modified proteins and wild-type) obtained by direct N-terminal sequence analysis of the purified virions. From these data, the number of copies of the gVIIIp displaying the p23 and RT2 peptides was calculated to approach respectively 10% and 30% of the total, both for phages single and double display.

### Primary cultures for generation of cytotoxic T cells

PBMC from the venous blood of HLA-A2 positive donors: LDA (HLA A2, DR 5,6) and MNO (HLA A2, DR7) were prepared. The cells were resuspended in RPMI 1640 and allowed to adhere in plates containing 2% gelatine at a density of 10⁶/cm². The adherent cells were separated from non adherent cells and used as APC. For this purpose they were irradiated (4000 rad) and 3x10⁵ cells pulsed for 3 hours at 37°C with the indicated antigens at the concentration of 20 µg/ml for peptides and 50 µg/ml for phages respectively. After 2 washes with RPMI the cells were mixed with 1x10⁶ non-adherent cells as a source of T lymphocytes and cultured in RPMI supplemented with 10% human autologous serum. Human recombinant IL2 (Boehringer, Germany) was added to the cultures after 3 days at the concentration of 10 U/ml. Anti-CD4 (Leu3a, IgG1, Becton Dickinson) or anti-HLA-DP (B7/21, IgG1, Becton Dickinson), used as control, were added to pulsed APC at the concentration of 10 µg/ml. Anti-CD40 activating mAb 14G7 (18) and anti-CD40 blocking mAb 5D12 were added at the concentration of 10 µg/ml.

### Cytotoxicity assay

Following 9 days culture, the ability of growing T cells to lyse ⁵¹ Cr-labelled-T2 target cells (a TAP -/-, HLA A2.1⁺, class II⁻ cell line) pulsed or unpulsed with 20 µg/ml pRT2 peptide were studied. Anti-CD4 (Leu3a, Becton Dickinson) and anti-CD8 (Boehringer, Mannheim) monoclonal antibodies were added to cultures set for cytolytic assay at the concentration of 50 µg/ml. The percentage of specific lysis was calculated as follows: 100 x (c.p.m. experimental release - c.p.m. spontaneous release)/(c.p.m. maximum release - c.p.m. spontaneous release). Spontaneous release was always < 20%.

### Results

9 days primary cultures were established to analyse the immunogenicity of phage particles carrying RT2 (ILKEPVHGV), a cytolytic HLA-A2 restricted peptide from HIV 1 reverse transcriptase (9). Adherent cells, to be used as presenting cells, were separated from the PBMC of two healthy individuals (LDA and MNO) by adsorption onto gelatine coated plates and pulsed with synthetic peptides, with phage constructs carrying single helper or cytolytic epitope or with constructs simultaneously displaying helper and cytolytic epitopes. The generation of HLA-A2 restricted cytotoxic T lymphocytes was then determined using, as a target, T2 cells (unable to present endogenously processed peptides because of a deletion of the Tap1 and Tap2 genes) loaded with the RT2 peptide. The results of Figure 1 show that cultures grown in the presence of peptide RT2 or phage constructs expressing the RT2 sequence do not generate RT2 specific CTL. In contrast, when cultures were performed in the presence of phages displaying both the cytolytic RT2 determinant and the T helper epitope pep23 also from HIV-1 RT (10,11), specific cytotoxic T cells were induced.

Furthermore, pep23 (KDSWTVNDIQKLVGK, corresponding to the immunodominant 249-263 sequence of RT) inserted into the gVIIIp phage major coat protein is recognised by specific CD4⁺ HLA-DR5 restricted T cell clones and how processing of phage particles is required.

An epitope inserted in the phage capsid can also induce class I restricted cytotoxic T cells, thus indicating that phage particles can enter the processing pathway where class I restricted epitopes are generated.

To check whether the RT2 and the pep23 determinants must be co-expressed on the surface of the same presenting cell in order to prime a specific CTL response, APCs were separately pulsed with either phage, washed and then mixed in 1:1 ratio so to obtain a population of presenting cells in which the two determinants are expressed onto the surface of different cells. As a control, adherent presenting cells were instead pulsed with a mixture of the RT2 and pep23 phages particle, respectively. Figure 2 shows that cytotoxic T cells were obtained when the presenting cells were pulsed with a 1:1 mixture of phages each displaying a single T helper or T cytolytic determinant. In contrast, when APC were separately pulsed with single displaying phages and mixed only later, induction of cytotoxicity was remarkably lower. These experiments clearly show, in agreement with previous reports (4,12,13), that priming of cytolytic T cells requires co-expression of the two determinants on the APC surface. It appears from these results that phage proteins do not contain determinants which may act as efficient DR5-restricted helper epitopes. Cytotoxic activity was negligible when target T2 cells were pulsed with pep789 (CTLSTVQLV), a control peptide carrying an HLA-A2 binding motif (14). Cultures were also established in the presence of the synthetic RT2 plus pep23. A much lower level of cytotoxic T cells activity was obtained, although the molar amount of peptide used was 3.5 fold in excess of the amount of peptide supplied as phages (Fig 2).

As pep23 is a promiscuous epitope which can be presented also in a DR7 restricted manner and the ability of double display phages to raise cytolytic activity was determined upon *in vitro* immunisation of PBMCs from an individual carrying the HLA-A2, HLA-DR7 haplotype (MNO); the same results of Fig. 1 and 2 were observed when MNO PBMCs were used.

To dissect the involvement of CD4 and CD8 T cell subsets in the lytic activity, experiments were performed in the presence of anti-CD4 and anti-CD8 mAbs. Addition of anti-CD8 mAb to the cytotoxic assay blocked lytic activity against RT2 loaded T2 cells of CTLs raised by priming with combined helper and cytolytic epitopes. In contrast an anti-CD4 mAb supplied at the same concentration to the mixture of effector and target T2 cells pulsed with RT2 peptide had a negligible inhibitory effect on cytotoxicity (Fig. 3a). These results indicate that the lytic activity is exerted by CD8+ T cells. However, CD4+ T cells play an essential role for the priming of a cytotoxic T cell activity. In fact, as shown in Figure 3b, an anti-CD4 mAb supplied when primary cultures were established blocked the generation of cytotoxic T cells, while a control antibody had a much reduced effect.

It was recently reported in various murine systems that signalling through CD40 on antigen presenting cells can replace the requirement for T helper cells (15-17). Cultures were thus established by pulsing adherent cells with phage particles in the presence of the anti-CD40 14g7 activating mAb or the anti-CD40 5D12 blocking mAb (18,19). As shown in Fig. 4, addition of the latter mAb significantly inhibited priming of RT2-specific T cells by adherent PBMC pulsed with a mixture of fd23 and fdRT2 phage particles, indicating that priming is dependent on CD40 activation. In the presence of the 14g7 activating mAB, instead, an increased cytolytic activity is observed upon priming with adherent PBMC pulsed with the fd RT2 phage alone (figure 4).

In conclusion, phage particles expressing both the pep23 and RT2 determinants are able to generate specific cytotoxic T cells *in vitro.* In addition, the cytolytic epitopes expressed on the surface of phage particles are powerfully immunogenic as they are able to elicit a specific cytotoxic T cell response much stronger than that induced by ten-fold molar excess of synthetic peptides (Fig. 2). The sequences chosen (pep23, aa 249-263 of HIV-1 reverse transcriptase and RT2, aa 309-317 of the same HIV-1 protein) derive from the "thumb" region of the polymerase domain of the HIV-1 RTase (20); both sequences show a high degree of conservation in different viral isolates (10). The pep 23 sequence includes not only a helper epitope, as shown, but does also contain a B cell epitope; neutralising antibodies against this determinant, inhibiting RT polymerase activity, were found in sera of long term non progressor HIV-1 infected patients and shown to correlate with survival (21). In addition, HLA-A2 HIV-1+ patients have been shown to develop at high frequency circulating CTL directed against a few Gag and Pol epitopes (among which the RT2 determinant used here) and correlating with protection parameters (22). The phage construct described above induces a specific anti-HIV-1 response.

Finally, while the use of synthetic peptides as immunogens finds obstacles because of the low antigenicity of these preparations (and consequent need of adjuvant) and because of stability and toxicity problems, the choice of phage particles appears to be promising. Phages in fact are not only much more efficient than peptides for generation of cytotoxic T cells, but in addition allow co-expression of multiple epitopes in the same cell. Phage particles do not require Freund's adjuvant for in vivo immunisation (7, 5), are non pathogenic, are unable to replicate in higher organisms and are non-toxic when injected in an animal model system. Furthermore, bacterial viruses are already used as marker vaccines for human applications (23). Multiple display *fd* phage particles thus appear ideally suited for the design of subunit vaccines.

### Example 2

### Cells and culture conditions

EBV-transformed B lymphocytes were established from a healthy donor, LD, with appropriate HLA class I and class II haplotypes (HLA DR 11, 13; DRB52: HLA-A2) and used as APC. Cells were cultured in RPMI 1640 with glutamax-1 (GIBCO Laboratories, Paisley, England) supplemented with 10% heat-inactivated foetal calf serum (PAA Laboratories, Linz, Austria), 100 U/ml penicillin G and 100 µg/ml streptomycin. PBMC from the same donor were separated by Ficoll/Paque centrifugation and resuspended in RPMI 1640 medium (Gibco) supplemented with 10% autologous plasma, 2 mM L-glutamine for further treatment to induce CTL responses (see below).

### Antibodies, peptides and reagents

Rabbit polyclonal antibodies against endoplasmic reticulum (ER), gift from D. Louvard (Institut Curie, Paris, France), and bovine mannose-6-phosphate receptor (MPR), gift from B. Hoflack (Institut Pasteur, Lille, France), monoclonal antibody (mAb) specific for lysosome associated membrane protein-1 (Lamp-1) (PharMingen, San Diego, CA), and mAb specific for the bacteriophage fd minor coat protein pIII were used to label ER, MPR compartments, lysosomes and the endocytosed phage, respectively. Phycoerythrin (PE)-conjugated mAb specific for HLA-DM molecules and the W6/32 mAb specific for HLA-class I (A, B, C) molecules were purchased from PharMingen (San Diego, CA) and Dako (Copenhagen, Denmark), respectively. Secondary polyclonal antibodies from commercial source were: PE- and Tricolor (TC)-conjugated goat anti-mouse IgG (GAM) and PE-conjugated goat anti-rabbit IgG (GAR) (Caltag Laboratories, Burlingame, CA). Markers specific for recycling compartments were tetramethylrhodamine isothiocyanate (TRITC)-conjugated transferrin, as previously described (30) 1996) and TRITC-conjugated dextran (Sigma Co., St Louis, MO).

Chemicals were purchased from Sigma, paraformaldehyde from Serva-Feinbiochemica (Heidelberg, Germany). Bacteriological media were from Difco. Plasmids (pfd8SHU and pTfd8SHU) and bacteriophages (fd, fdAMPLAY88 and fdISPLAY8) have been described previously (Malik et al., 1996; Malik and Perham, 1997).

### Construction of hybrid bacteriophage virions

Hybrid bacteriophage virions, fd23, displaying peptide p23 (KDSWTVNDIQKLVGK) and the double-display hybrid, fd23RT2, displaying both p23 and peptide RT2 (ILKEPVHGV), were constructed. An oligonucleotide sequence encoding p23 (KDSWTVNDIQKLVGK) was cloned into plasmid pfd8SHU to create plasmid pfd8p23. *E. coli* TG1 *recO* cells transformed with pfd8p23 were infected with the bacteriophage fdISPLAY8 to generate bacteriophage fd23. The double-display bacteriophage, fd23RT2, was constructed by cloning an oligonucleotide sequence encoding RT2 (ILKEPVHGV) into bacteriophage fdAMPLAY88 to generate bacteriophage fdAMPLAY88-RT2. The simultaneous display of peptides 23 and RT2 on the same hybrid virion was achieved by transforming *E. coli* RG1 *recO* cells with plasmid pTfd8p-66 (Malik and Perham, 1997), which encodes pep23, and then infecting them with fdAMPLAY88-RT2. The hybrid virions were harvested, purified and analysed by SDS-PAGE, and submitted to N-terminal amino acid sequence analysis, all as described previously (Malik et al., 1996).

### Fluorescent labelling of bacteriophage virions

Wild-type bacteriophage fd virions (1-3 mg / ml) in 50 mM sodium phosphate buffer, pH 9.0, containing 0.15 M NaCl were treated with a ten-fold molar excess of fluorescein isothiocyanate (FITC) (based on the molarity of the pVIII protein, *M*ᵣ 5239, in the sample). The mixture was stirred gently for 2 hours at room temperature and unreacted FITC and other small molecules were then removed by gel filtration on a PD10 (Sephadex G25) column (Pharmacia, Uppsala, Sweden) in PBS, pH 7.4. The incorporation of fluorescent fluorescein thiocarbamyl (FTC) groups was estimated by SDS-PAGE analysis (Malik et al., 1996) and MALDI mass spectrometry (Terry et al., 1998). It is estimated that 10% of the pVIII molecules in the virion had been modified under these conditions. The measured mass of the modified protein (5628 Da) compared with that of the unmodified protein (5239) indicated that the modified protein had incorporated only one FTC group (expected mass difference 389 Da). Based on previous studies of the chemical modification of the bacteriophage fd virion (Armstrong et al., 1983), this is likely to be the N₆-amino group of Lys8.

### Confocal microscopy analysis and intracellular localisation of FITC-conjugated fd phage

EBV-B lymphocytes, LD (1 x 10⁶/ml) were cultured in 24-well plates (Nunc, Roskilde, Denmark) at 37°C in a 5% CO₂ atmosphere with FITC-conjugated fd phages (20 µg/10⁶ cells) for various times, harvested, washed twice in cold PBS and resuspended in PBS at 0°C. Cell viability was controlled by addition of 5 µg/ml propidium iodide (PI), immediately before observation. For endosome labelling, LD cells previously cultured with FITC-conjugated fd phages for various times, were further incubated in culture medium at 37 °C with TRITC-conjugated transferrin (100 nM) or with TRITC-conjugated dextran (3 mg/ml) for 5 to 20 min or 1h, respectively. For intracellular staining, cells were fixed in 500 µl 4% paraformaldehyde and 0.025% glutaraldehyde in PBS for 30 minutes on ice and were successively treated with 500 µl 0.1% Na borohydride in PBS and with 50 mM NH₄Cl in PBS for 10 min at room temperature. Permeabilisation was achieved by a 30 min incubation at room temperature in PBS containing 0.05% saponin. Indirect immunofluorescence was performed by successive 30 min incubations at room temperature of LD cells with primary mAbs or polyclonal antibodies, specific for the different cellular markers, and with PE- or TC-conjugated secondary antibodies diluted in PBS containing 0.05% saponin. After washing, cells were pelleted by 1400 rpm centrifugation for 10 min. Pellets were resuspended in 5 µl Mowiol containing 2.5% DABCO (Sigma) and put onto microscope glass slides, covered with cover glass and dried for 2 h at room temperature. Labelled specimen were scanned with an ACAS 570 Interactive Laser Cytometer (Meridian Instruments, Inc., Okemos, MI), equipped with confocal optics. The system consisted of a 5 W argon ion laser tuned to 488 nm, an Olympus IMT-2 inverted microscope with a 100x oil immersion objective (N.A.1.3) Z-axis control, an XY scanning stage, and a variable pinhole aperture, all under 80486 computer coordination. The ACAS 570 was set up in fluorescence mode. FITC, TC, TRITC and PE were excited at 488 nm. The computer converts fluorescence intensity into colour as output image. The excitation was done at 20 mW power and the filter constitution for emission detection was the conventionally used for FITC 520 nm BP, and TC 670 nm BP or TRITC/PE 580 BP. To have a good resolution, pinhole and photomultiplier were set at 40% and 25%, respectively. Sections were taken every 1 µm and the images shown are the calculated projections of all sections. The pictures obtained are representative of over 100 cells analysed from two independent experiments, at least.

### CTL priming and cytotoxicity assays

CTL priming was performed as previously described (De Berardinis et al. 1999, in preparation). Briefly, peripheral blood T lymphocytes and monocytes isolated from donor LD were cultured for 9 days with double hybrid phage fd23RT2. CD8+ T cells were harvested and assayed for cytotoxic activity. EBV-transformed B cells LD (1 x 10⁶) were used as target cells and were incubated with recombinant phage fdRT2 (50 µg/ml) or with synthetic peptides RT2 or 789 (20 µg/ml) overnight in RPMI without serum. Cells were labelled with 100 µCi of Na ⁵¹chromate (Amersham) for 90 min at 37 °C, washed, and diluted to 5 x 10³ cells/well in 96-well microtiter plates. Serial dilutions of primed CD8+ T cells were added to the assay wells. After a 6 h incubation, ⁵¹Cr release in the supernatant was measured in a gamma counter (Beckmann). Spontaneous release was determined in wells with target cells only and maximum release was determined by target cell lysis with a detergent (5% NP40). The specific lysis was calculated as follows: 100x d.p.m. (Experimental Release-d.p.m. Spontaneous Release) / (d.p.m. Maximum Release - d.p.m. Spontaneous Release).

### RESULTS

### Uptake of wild-type bacteriophage fd in APCs

To define the fate of ingested virions in APCs, bacteriophage fd virions were treated with fluorescein isothiocyanate. The fluorescent virions (FITC-fd) retained their structural integrity and, as judged by SDS-PAGE and mass spectrometry, exhibited modification of about 10% of the 2700 pVIII coat proteins per virion. Only one FTC derivative per pVIII protein was detected by mass spectrometry and, based on previous studies of the chemical modification of bacteriophage fd (Armstrong et al. (1983)), it was suspected that Lys8 is the site of modification.

B-_{EBV} lymphocytes, LD, established from peripheral blood of a healthy volunteer were used as APCs. This cell line derives from the same donor who gave rise to T helper cell lines (29) and CTL cells specific for HIV-1 RT-derived Th (p23) and CTL (RT2) epitopes, respectively. B cells were incubated with FITC-*fd* in culture medium at 37°C for various times. After 1 hour, FITC-*fd* was detectable in a few cells. A time-course study indicated that a 9 h to an overnight incubation yielded an optimal uptake of FITC-*fd* by most cells. Optical sections with confocal laser microscope confirmed the intracytoplasmic localisation of FITC-*fd*. The uptake of FITC-*fd* did not induce cell death as controlled by the lack of nuclear staining with propidium iodide, indicating that FITC-*fd* internalisation had no cytotoxic effect.

### Intracellular degradation of FITC-fd virions requires an overnight incubation

To assess the integrity/degradation of FITC-*fd* particles internalised in B lymphocytes, the binding of a mAb specific for the pIII minor coat protein of the bacteriophage was detected with a TC-conjugated secondary antibody. B cells incubated for 9 h with FITC-*fd* exhibited a colocalisation of the two fluorochromes, indicating that most FITC-*fd* virions were intact. Conversely, after an overnight incubation most B cells appeared green coloured suggesting that fragmentation of FITC-*fd* had occurred.

### Wild-type bacteriophage fd enters both the MHC class I and class II processing pathways

To determine the fate of FITC-*fd* virions inside B-_{EBV} lymphocytes, colocalisation of FITC-*fd* fragments and TRITC-labelled markers specific for different intracellular compartments were studied and analysed under confocal microscope. The endosomal compartments were labelled with TRITC-conjugated transferrin or dextran, which are specific for the recycling pathway. When incubated for 5 or 20 min with the cells, transferrin is localised in early or late endosomes, respectively; when incubated for 1 h, dextran is localised in late endosomes. In B-_{EBV} lymphocytes cultured for 9 h with FITC-*fd*, incubation with TRITC-transferrin for 5 min resulted in a weak colocalisation of the 2 fluorochromes, whereas a 20 min incubation yielded a yellow staining of most cells. This suggests that the *fd* virions had already left early endosomal compartments and were localised in late recycling endosomes. This was confirmed by the colocalisation of FITC-*fd* virions and TRITC-conjugated dextran, incubated for 1 h with B cells. Conversely, in B cells cultured for 20 h with FITC-*fd* virions, the localisation of phage fragments in late endosomes was considerably reduced as indicated by the poor colocalisation observed with TRITC-conjugated dextran in a few cells, suggesting that phage fragments already left this compartment. In fact, at this time FITC-*fd* fragments were found in pre-lysosomal and lysosomal degradation compartments, as indicated by their colocalisation with TRITC-conjugated antibodies directed against MPR and Lamp-1, respectively.

To establish whether FITC-*fd* fragments were addressed to MHC class II peptide loading compartments, B-_{EBV} lymphocytes cultured for 20 h with FITC-*fd* virions, were stained with a PE-conjugated anti-HLA-DM mAb. Most cells were yellow coloured, indicating that FITC-*fd* fragments colocalise with HLA-DM molecules. More interestingly, FITC-*fd* fragments were also found in the ER, as detected with an anti-ER antibody. Conversely, no colocalisation was observed with any of these markers in B-_{EBV} lymphocytes incubated for 9 h with FITC-*fd* virions. In all cases, optical sections confirmed that FITC-*fd* fragments and TRITC-labelled specific markers were confined to B lymphocyte cytoplasm. These results indicate that after their uptake by APC *fd* virions can be targeted to both class I and class II processing pathways.

### Specific lysis of EBV-B target cells by CTL primed with hybrid phages

The EBV-B cell lines are able to process *fd* phages expressing helper epitopes and to present them to class II restricted CD4+ T cells. Furthermore, the CTL epitope RT2 displayed by hybrid phages fdRT2 can also be correctly processed by B-EBV lymphocytes from donor LD and be recognised by HLA-A2 restricted CD8+ CTL cells. HLA-A2 restricted CTL were induced by priming human PBL from donor LD with double hybrid phage particles fd23RT2. Only double hybrid phage and not single hybrid or wild type phage were able to induce CTL production. The specificity of CTL was assayed in a ⁵¹Cr release assay using as target cells the B-EBV cell lines pulsed with bacteriophage fdRT2. As illustrated in Figure 5A, B-EBV target cells pulsed with fdRT2 virions were killed by specific CTL while unpulsed B-EBV or B-EBV pulsed with fd23 virions were not lysed. Specificity of recognition was further confirmed by cytotoxicity assay using as target cells B-EBV respectively pulsed with RT2 synthetic peptides or an irrelevant peptide known to bind HLA-A2 (Fig. 5B). These results indicate that B lymphocytes are able to correctly process fdRT2 and to present the RT2 epitope to appropriate class I molecules.

### Example 3

### In vivo results with the double hybrid bacteriophage according to the invention

### Animals and immunisation conditions

Having demonstrated that phage particles were able to induce specific cytotoxic T cells in *in vitro* cultures generated from human PBL isolated from HLA-A2+ donors, the inventors assayed to extend these observations to an "*in vivo"* system. For this purpose, HLA-A2 transgenic mice, kindly provided by Dr. F. Lemonnier (Institut Pasteur, Paris, France) and previously described (45), were immunised subcutaneously as previously described (46) (at the bottom of the tail with 140 µg of either single or double displaying phage particles in PBS, in presence or absence of an equal volume of Freund's adjuvant). In a group of mice this protocol of immunisation was repeated weekly for three times.

### CTL priming and cytotoxicity assays

The induction of specific cytotoxic cells was analysed in the spleen of immunised mice after 1-3 cycles of immunisation as previously described (45). Briefly 7 days after the last immunisation, the mice were sacrificed and the isolated splenocytes were cultured with antigen pulsed LPS blasts produced from syngeneic not-immunised animals. LPS blasts were obtained by culturing for 3 days the splenocytes in RPMI 10% FCS, 2me 5.10⁻⁵M, glutamine 1 mM, sodium pyruvate 1 mM, LPS 25 µg/ml, sulfate de Dextran 7 µg/ml. these cells were then pulsed with phage particles expressing the relevant antigen. Pulsing was performed by incubating 30 x 10⁶ cells/ml with 50 µg/ml of phage particles 3 hours at 37 °C. At the end of this incubation period the cells were washed, γ-irradiated (10000 rads). finally, 2.5 x 10⁶ LPS blasts were cultured with 5 x 10⁶ splenocytes from immunised mice in 2 ml of culture medium. After 5 days cytotoxic activity of cultured cells was analysed in a 6 hours ⁵¹Cr release assay. As target of cytotoxicity RMA^{-S} cells (murine analogous of T2 cells) transfected with HLA-A2 molecules were used.

### Results : Induction of specific CTL in immunised animals

As shown in Fig. 6A specific cytolytic activity was detected after 1 immunisation on splenocytes isolated from mice immunised with phage particles in the presence of Freund's adjuvant. However, after three immunisations, the phage particles were able to induce CTL production also in the absence of Freund's adjuvant (Fig. 6B). In contrast to data obtained *in vitro,* immunisation with fdRT2 phage particles (not expressing the helper epitope pep23) was able, indeed, to elicit the generation of specific CTL. The inventors have identified in the phage carrier protein gVIIIp the presence of an epitope which is recognised by murine CD4+ T cells and which may provide the help for CTL induction.

### REFERENCES

1) Bona, C. A. et al., *Immunol. Today* **19**, 126-133 (1998).
2) Demotz, S. et al., *Science* **249,** 1028-1030 (1990).
3) Bot, A. et al., *J. Immunol.* **157,** 3436-3442 (1996).
4) Stuhler, G. & Schlossman, *Proc.Natl.Acad.Sci.USA* **94,** 622-627 (1997).
5) Greenwood, J. et al., *J. Mol. Biol.* **220,** 821-827 (1991).
6) Minenkova, O. O. et al., *Gene* **128,** 85-88 (1993).
7) Di Marzo Veronese, F. et al., *J. Mol. Biol.* **243**, 167-172 (1994).
8) Marvin, D. A. et al., *J.Mol.Biol.* **235**, 260-286 (1994).
9) Walter, J. B. et al., *Int. Immunol.* **9,** 451-459 (1997).
10)Meyers, G. et al., *Human Retroviruses and AIDS* (Los Alamos National Laboratory, Los Alamos, NM, 1993).
11)Manca, F. et al., *Eur.J.Imunol.* **26**:2461-2469 (1996).
12)Ossendorp, F. et al., *J.Exp.Med.* 187, 693-672 (1998).
13)Keene, J & Forman, J., J. *Exp. Med.* **155,** 768-782, (1982).
14)Di Modugno, F. et al., J. *Immunotherapy* **20**, 431-436 (1997).
15)Ridge, J. P. et al., *Nature* **393,** 474-478 (1998).
16)Bennett, S. R. M. et al., *Nature* **393,** 478-480 (1998).
17)Schoenberger, S. P. et al., *Nature* **393,** 480-483 (1998).
18)
19)de Boer, M. et al., *Eur.J.Immunol.* **23:** 3120-3125 (1993).
20)Ren, J. et al., *Nat. Struct. Biol.* **4,** 293-302 (1995).
21)Grimison, B. & Laurence, J., *J.Acquir.Immune Defic. Syndr. Hum.Retrovirol.* **9,** 58-68 (1995).
22)Ogg, G.S. et al., *Science* **279,** 2103-2106 (1998).
23)Ochs, H.D. et al., *Blood* **80,** 1163-71 (1992).
24)Malik, P. & Perham, *Gene* **171,** 49-51 (1996).
25)Malik, P. et al., Multiple Display of Foreign Peptides Epitopes on Filamentous Bacteriophage Virions. In *Phage Display of Peptides and Proteins* (eds Kay, B.K., Winter, J. and McCafferty, J.) (Academic Press, San Diego, 1996).
26)Malik, P., & Perham R.N., *Nucleic Acids Res.* **25,** 915-(1997).
27)Chapman, H. A., *Curr. Opin. Immunol.* **10,** 93-100 (1998). 28)De Berardinis, P. et al., *Human Immunol.* **54,** 189-193 (1997).
29)De Berardinis, P. et al., *Vaccine* **17,** 1434-1441 (1999).
30)Durrbach, A. et al., *J. Cell Sci.* **109,** 457-465 (1996).
31)Germain, R. N., *Cell* **76,** 287-299 (1994).
32)Germain, R. N., & Margulies, D. H., *Annu. Rev. Immunol.* **11**, 403-450 (1993).
33)Geuze, H. J., *Immunol. Today* **19,** 282-287 (1998).
34)Iannolo, G. et al., *J. Mol. Biol.* **248**, 835-844 (1995).
35)Jelinek, R. et al., *J. Mol. Biol.* **266,** 649-655 (1997).
36)Malik, P. et al., *J. Mol. Biol.* **260,** 9-21 (1996).
38)Pamer, E., & Cresswell, P., *Annu. Rev. Immunol.* **16,** 323-358 (1998).
39)Parmley, S. F., and Smith, G. P., *Gene* **73,** 305-318 (1988).
40)Perham, R. N. et al., *FEMS Microbiol. Rev.* **17**, 23-31 (1995).
41)Pieters, J., *Curr. Opin. Immunol.* **9,** 89-96 (1997).
42)Rock, K. L., *Immunol. Today* **17,** 131-137 (1996).
43)Shastri, N. et al., *Curr. Opin. Immunol.* **10,** 137-144 (1998).
44)Smith, G. P., *Science* **228,** 1315-1317 (1985).
45)Pascolo et al., *J. Exp. Med.,* Vol. 185(12), p. 2043-2051 (1997).
46)Bennet et al., *Nature,* Vol. 393, p. 478-480 (1998).

## Claims

1. A double hybrid filamentous bacteriophage engineered to display at least a CTL epitope.

2. A filamentous bacteriophage according to claim 1, engineered to further display either a Th cell epitope or a Th cell and B cell epitope.

3. A filamentous bacteriophage according to the claim 1 or 2, engineered in the major coat (pVIII) protein.

4. A filamentous bacteriophage according to any of the claims 1 to 3, wherein said epitope is able to raise an immune response against an infectious agent or against a tumoral cell.

5. A filamentous bacteriophage according to claim 4, characterised in that said infectious agent is selecting from the group consisting of viral or bacterial agents or parasites.

6. A filamentous bacteriophage according to claim 5, characterised in that the viral infectious agent is the HIV virus.

7. A filamentous bacteriophage according to claim 5, wherein said infectious agent is plasmodium.

8. A filamentous bacteriophage according to any of the preceding claims, wherein the CTL epitope comprises the following amino acid sequence: SEQ ID n°1:ILKEPVHGV.

9. A filamentous bacteriophage according to any of the preceding claims 2 to 8, wherein the Th cell and B cell epitope comprises the following amino acid sequence: SEQ ID n°2:KDSWTVNDIQKLVGK.

10. A pharmaceutical composition comprising a pharmaceutically adequate carrier and the double hybrid filamentous bacteriophage according to any of the preceding claims 1 to 9.
